# EUROPEAN PATENT APPLICATION

(11) **EP 4 692 324 A1**
(43) Date of publication of application: **11.02.2026**
(21) Application number: 24781352.0
(22) Date of filing: 14.03.2024
(51) Int. Cl.: C12N 5/078

(54) **METHODS FOR PRODUCING RED BLOOD CELLS AND PLATELETS**

(30) Priority: 29.03.2023 KR 20230041264; 13.03.2024 KR 20240035092
(71) Applicant: Dewcell, Inc., Seongnam-si, Gyeonggi-do 13494 (KR)
(72) Inventor: CHO, Yeon Hee, Seoul 06370 (KR); KIM, Chi Hwa, Gwangju 61991 (KR)
(74) Representative: Klöckner, Christoph
(86) International application number: PCT/KR2024/095518
(87) International publication number: WO 2024/205363

(57) **Abstract**

The present invention relates to a method for producing red blood cells, more particularly, a method capable of producing red blood cells of excellent quality in a short period of time, which includes: (S1) culturing pluripotent stem cells to obtain a culture medium including hematopoietic stem cells; (S2) obtaining a CD41⁻/CD34⁻ cell population from the culture medium; and (S3) maturing the obtained cell population in a first medium including erythropoietin (EPO).

## Description

### [Technical Field]

The present invention relates to a method for producing red blood cells and/or platelets.

### [Background Art]

Blood transfusion is a very important treatment for patients suffering from a blood deficiency due to various causes.

However, the number of blood donors is decreasing due to the aging population and the decrease in the number of blood donors. Further, the recent spread of infectious diseases such as Covid-19 has led to an extreme blood supply shortage.

The supply of blood from donors involves the risk of transmission of infectious diseases and other transfusion-related adverse effects. Therefore, the stable supply of red blood cells and platelets is an important task in the corresponding technical field.

There have been attempts to use hemoglobin solutions or oxygen carriers as alternatives to red blood cells, which are used for oxygen transport purposes among blood components, but they have shown low oxygen transport capacity or serious side effects. Therefore, it is required to produce red blood cells and platelets that can eliminate immunological side effects and the risk of infection.

In order to meet this demand, studies have been conducted recently to produce red blood cells and platelets in vitro. However, conventional red blood cell or platelet production methods have the problem that they take a long time for production and do not produce the product with a high yield. In addition, since these materials must be produced individually, the process efficiency and economic efficiency are low. Therefore, technological improvement is still needed for an economical method of producing artificial red blood cells and platelets that can be mass-produced.

### [Summary of Invention]

### [Problems to be Solved by Invention]

It is an object of the present invention to provide a method capable of producing red blood cells in a short period of time.

It is another object of the present invention to provide a method capable of producing red blood cells with a high yield.

It is yet another object of the present invention to provide a method for simultaneously producing red blood cells and platelets.

It is still another object of the present invention to provide a method for producing red blood cells with high process efficiency.

It is a further object of the present invention to provide a method for simultaneously producing red blood cells and platelets with high process efficiency.

### [Means for Solving Problems]

1. A method for producing red blood cells, including: (S1) culturing pluripotent stem cells to obtain a culture medium including hematopoietic stem cells; (S2) obtaining a CD41⁻/CD34⁻ cell population from the culture medium; and (S3) maturing the obtained cell population in a first medium including erythropoietin (EPO).
2. The method according to the above 1, further including: proliferating the obtained cell population in a second medium including erythropoietin, stem cell factor, and interleukin-3.
3. The method according to the above 1, further including: differentiating the obtained cells in a third medium including erythropoietin and stem cell factor.
4. The method according to the above 1, wherein the culture medium includes red blood cell precursors and megakaryocyte precursors.
5. The method according to the above 1, wherein the maturation step is performed for 4 to 14 days.
6. The method according to the above 1, wherein (S1) includes: (S1a) culturing pluripotent stem cells in a fourth medium including a GSK3 inhibitor; (S1b) culturing the cells cultured in the fourth medium in a fifth medium including vascular endothelial growth factor and basic fibroblast growth factor; and (S1c) culturing the cells cultured in the fifth medium in a sixth medium including vascular endothelial growth factor, basic fibroblast growth factor, and a transforming growth factor beta signaling inhibitor.
7. The method according to the above 1, further including: (S0) seeding pluripotent stem cells at a density of 2,000 to 20,000 cells/cm² on the bottom of a culture vessel
8. The method according to the above 1, wherein the pluripotent stem cells include human induced pluripotent stem cells.
9. A method for producing a blood product, including a process of mixing red blood cells produced by any one of the methods 1 to 8 above with other blood components.
10. A method for producing red blood cells and platelets, including: (S1) culturing pluripotent stem cells to obtain a culture medium including hematopoietic stem cells; (S2) sorting a CD41⁻/CD34⁻ cell population and a CD41a⁺ or CD34⁺ cell population from the culture medium; (S3) maturing the sorted CD41⁻/CD34⁻ cell population in a first medium including erythropoietin; and (S4) differentiating the sorted CD41a⁺ or CD34⁺ cell population into megakaryocytes in a seventh medium.
11. The method according to the above 10, further including: proliferating the sorted CD41⁻/CD34⁻ cell population in a second medium including erythropoietin, stem cell factor and interleukin-3.
12. The method according to the above 10, further including: differentiating the sorted CD41⁻/CD34⁻ cell population in a third medium including erythropoietin and stem cell factor.
13. The method according to the above 10, wherein the culture medium includes red blood cell precursors and megakaryocyte precursors.
14. The method according to the above 10, wherein (S3) is performed for 4 to 14 days.
15. The method according to the above 10, wherein (S1) includes: (S1a) culturing the pluripotent stem cells in a fourth medium including a GSK3 inhibitor; (S1b) culturing the cells cultured in the fourth medium in a fifth medium including vascular endothelial growth factor and basic fibroblast growth factor; and (S1c) culturing the cells cultured in the fifth medium in a sixth medium including vascular endothelial growth factor, basic fibroblast growth factor, and a transforming growth factor beta signaling inhibitor.
16. The method according to the above 10, further including: seeding the pluripotent stem cells (S0) at a density of 2,000 to 20,000 cells/cm² on the bottom of a culture vessel.
17. The method according to the above 10, further including: culturing and maturing megakaryocytes in an eighth medium including thrombopoietin.
18. The method according to the above 10, wherein the seventh medium includes thrombopoietin, stem cell factor, interleukin-3 and interleukin-6.
19. The method according to the above 10, wherein the pluripotent stem cells include human induced pluripotent stem cells.
20. A method for producing a blood product, including mixing red blood cells and platelets produced by the method according to any one of the above 10 to 19.

### [Advantageous effects]

The red blood cell production method of the present invention produces red blood cells from a CD41⁻/CD34⁻ cell population, and thus the period required for red blood cell production can be shortened.

The red blood cell production method of the present invention enables mass production of red blood cells because the CD41⁻/CD34⁻ cell population not only exhibits high differentiation potential into red blood cells but also exhibits high cell proliferation potential.

The red blood cell production method of the present invention has a simple process, does not require complex equipment, and is advantageous in terms of time and cost.

The method for producing red blood cells and platelets of the present invention is capable of simultaneously producing red blood cells and platelets, and therefore provides high process efficiency.

Red blood cells and platelets produced according to the method of the present invention can exhibit therapeutic or preventive effects for diseases requiring blood transfusion.

### [Brief Description of Drawings]

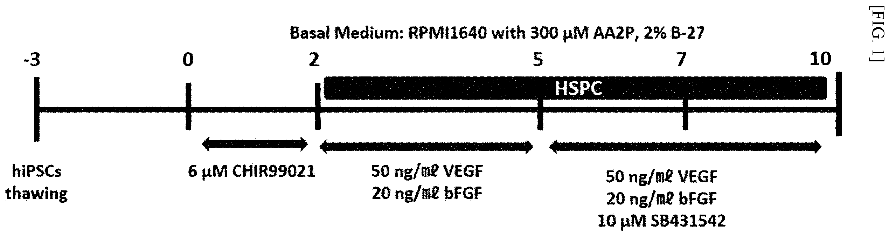

FIG. 1 is an example of a process for culturing human induced pluripotent stem cells.

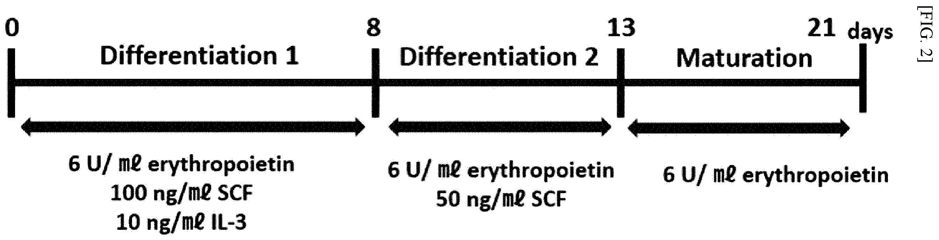

FIG. 2 is an example of a process for differentiating CD41a⁻/CD34⁻ cells into erythrocytes.
FIG. 3 shows the results of confirming the pellet color of the culture medium obtained in Preparative Examples 1 and 2 for production of red blood cells.
FIG. 4 shows the results of blood cell staining of cells obtained in Preparative Examples 1 and 2 for production of red blood cells.
FIG. 5 shows the results of immunostaining cells obtained in Preparative Examples 1 and for production of red blood cells.
FIG. 6 shows the results of FACS analysis of cells obtained in Preparative Examples 1 and 2 for production of red blood cells.
FIG. 7 shows the results of confirming the proliferation rate of cells obtained in Preparative Example 1 for production of red blood cells.
FIG. 8 shows the results of hemoglobin ELISA performed on cells obtained in Preparative Examples 1 and 2 for production of red blood cells.
FIG. 9 shows the results of observing cells obtained in Preparative Examples 1 and 2 for production of red blood cells using a transmission electron microscope.
FIG. 10 shows the results of observing cells obtained in Preparative Examples 1 and 2 for production of red blood cells using a scanning electron microscope.
FIG. 11 shows the results of confirming surface markers (CD34, CD45, CD41a) of the floating cell population in the floating cell preparative example and comparative preparative example.
FIG. 12 illustrates that the cell population in Floating Cell Preparative Example 1 expresses megakaryocyte-specific markers.
FIG. 13 illustrates that the cell population in Floating Cell Preparative Example 1 expresses megakaryocyte-specific markers.
FIG. 14 illustrates that the cell population obtained from Floating Cell Comparative Preparative Example 1 does not express megakaryocyte-specific markers.
FIG. 15 illustrates that cells obtained from Floating Cell Preparative Example 1 were activated by ADP treatment, resulting in increased expression of PAC-1 and CD62p.
FIG. 16 illustrates that cells obtained from Floating Cell Preparative Example 3 were activated by ADP treatment, resulting in increased expression of PAC-1 and CD62p.
FIG. 17 shows the results of selecting only CD41a⁺ cells from the cell population obtained in Floating Cell Preparative Example 1.
FIG. 18 shows the results of confirming the expression of platelet-specific markers in a cell population of selected CD41a⁺ cells and a cell population in Floating Cell Preparative Example 1.
FIG. 19 shows the results confirming that differentiating the cell population in Floating Cell Preparative Example 1 into platelets exhibits high cell proliferation capacity.

### [Mode for Carrying out Invention]

The present invention relates to a method for producing red blood cells or platelets.

The production method of the present invention may prepare red blood cells or platelets separately, or may produce red blood cells and platelets together.

The method for producing red blood cells of the present invention may include: (S1) culturing pluripotent stem cells to obtain a culture medium including hematopoietic stem cells; (S2) obtaining a CD41⁻/CD34⁻ cell population from the culture medium; and (S3) maturing the obtained cell population in a first medium including erythropoietin (EPO).

The red blood cell production method of the present invention may further include (S0) seeding pluripotent stem cells at a density of 2,000 to 20,000 cells/cm² on the bottom of a culture vessel.

The red blood cell production method of the present invention may further include proliferating the obtained cell population in a second medium including erythropoietin, stem cell factor, and interleukin-3.

The red blood cell production method of the present invention may further include differentiating the obtained cells in a third medium including erythropoietin and stem cell factors.

Hereinafter, the process for producing red blood cells will be described in detail.

(S0) of the red blood cell production method of the present invention is a step of seeding pluripotent stem cells at a density of 2,000 to 20,000 cells/cm² on the bottom of a culture vessel.

Pluripotent stem cells include induced pluripotent stem cells (iPSCs) or embryonic stem cells (ESCs).

Induced pluripotent stem cells (iPSCs) are differentiated cells that lack pluripotency and acquire pluripotent differentiation ability through an artificial reprogramming (dedifferentiation) process.

Induced pluripotent stem cells (iPSCs) may be derived from an organism selected from the group consisting of humans, non-human primates, rodents (mouse, rat), ungulates (cows, sheep, etc.), dogs (pet and wild dogs), cats (pet and wild cats such as lions, tigers, cheetahs), rabbits, hamsters, goats, elephants, pandas (including giant pandas), pigs, raccoon dogs, horses, zebras, and marine mammals (dolphins, whales, etc.).

### Induced pluripotent stem cells may be human induced pluripotent stem cells (hiPSCs)

Induced pluripotent stem cells may be generated using mouse and/or human cells. For example, induced pluripotent stem cells may be generated using embryonic, fetal, neonatal, and adult tissues.

Induced pluripotent stem cells may be used as a starting point and substantially from any somatic cell at any stage of development. The somatic cell may be, but is not limited to, derived from an embryonic, fetal, neonatal, juvenile, or adult donor. The somatic cell may be, but is not limited to, a fibroblast, such as a skin fibroblast obtained from a skin sample or biopsy, synoviocyte from synovial tissue, a buccal cell, or a lung fibroblast.

Pluripotent stem cells are seeded on the bottom of the culture vessel and then cultured under adherent conditions.

The incubator may be used without limitation as long as it is used for cell culture in the art. For example, it may be a large, medium, or small incubator. Further, it may be a cell culture flask such as a T25, T75, T175, or T225.

In terms of cell confluency, it is preferable that pluripotent stem cells are seeded at a density of 2,000 to 20,000 cells/cm². When seeded in this amount, the cell confluency may be 70% to 95%, 75% to 95%, 80% to 95%, or 85% to 95%, and the pluripotent stem cells may be appropriately cultured and differentiated, whereby a culture medium including a sufficient amount of hematopoietic stem cells, hematopoietic progenitor cells, and megakaryocyte progenitor cells can be obtained in the step (S1) described below.

When the pluripotent stem cells are seeded on the bottom of the culture dish at less than 2,000 cells/cm², the cell confluency may be 70% or less, 65% or less, 60% or less, 55% or less, 50% or less, 45% or less, 40% or less, 35% or less, 30% or less, etc. at the time of obtaining the floating cell population of (S2). Further, when the pluripotent stem cells are seeded on the bottom of the culture dish at more than 20,000 cells/cm², the cell confluency may exceed 100% at a time when the pluripotent stem cells are not sufficiently cultured and differentiated, therefore, undifferentiated cells may fall off the culture dish and remain in a floating state and die, or the amount of factors added to the culture medium may become insufficient, which may cause poor signaling between cells and poor differentiation.

Step (S1) of the red blood cell production method of the present invention is a step of culturing pluripotent stem cells to obtain a culture medium including hematopoietic stem cells. This step is a step of culturing pluripotent stem cells seeded in step (S0) to obtain a floating cell population capable of differentiating into red blood cells. The culture medium obtained in this step contains red blood cell precursors and megakaryocyte precursors.

Step (S1) may consist of the following sub-steps:
(S1a) culturing pluripotent stem cells in a fourth medium including a GSK3 (Glycogen Synthase Kinase 3) inhibitor;
(S1b) culturing the cells cultured in the fourth medium in a fifth medium including vascular endothelial growth factor (VEGF) and basic fibroblast growth factor (bFGF); and
(S1c) culturing the cells cultured in the fifth medium in a sixth medium including vascular endothelial growth factor; basic fibroblast growth factor; and a transforming growth factor beta signaling inhibitor.

The fourth, fifth, and sixth media used in this step are media with different compositions and purposes.

The fourth compound includes at least a GSK3 inhibitor. The GSK3 inhibitor may be, but is not limited to, CHIR99021, BIO (6-bromoindirubin-30-oxime), SB216763, CHIR-98014, CT98014, CT98023, CT99021, TWS119, SB41528, AR-A014418, AZD-1080, Alsterpaullone, Cazpaullone or Kenpaullone. CHIR99021 is a GSK3 inhibitor and a Wnt signaling agonist, and may be represented by an aminopyrimidine.

The GSK3 inhibitor (e.g., CHIR99021) is not limited to a specific concentration as long as it is an amount capable of culturing pluripotent stem cells. The GSK3 inhibitor may be included in the fourth medium at a concentration of, for example, 2 to 10 µM, 2.5 to 9.5 µM, 3 to 9 µM, 3.5 to 8.5 µM, 4 to 8 µM, 4.5 to 7.5 µM, 5 to 7 µM, 5.5 to 6.5 µM or 6 µM.

The fourth medium is a basal medium. The fourth medium may include RPMI1640 medium or other types of basal medium. The fourth medium may further include antioxidants and/or B-27 in addition to the basal medium.

Antioxidants may include any one selected from the group consisting of 6-hydroxymelatonin, acetyl-L-carnitine (ALCAR), alpha-lipoic acid (ALA), ascorbic acid (e.g., L-Ascorbic acid 2-phosphate (AA2P), L-Ascorbic acid 2-glucoside (AA2G), carotenoids (vitamin A), curcumin, edaravone, polyphenols, glutathione, hydroxytyrosol, L-carnitine, ladostigil, melatonin, mofegiline, N-acetylcysteine (NAC), N-acetylserotonin (NAS), oleocanthal, oleuropein, rasagiline, resveratrol, selegiline, selenium, tocopherols (vitamin E), tocotrienols, tyrosol, ubiquinone (coenzyme Q), and uric acid.

The fifth medium is a growth medium. The fifth medium may include at least growth factor such as vascular endothelial growth factor (VEGF) and basic fibroblast growth factor (bFGF). The fifth medium may further include other growth factors for culturing pluripotent stem cells.

Vascular Endothelial Growth Factor (VEGF) is a member of the Epidermal Growth Factor Receptor (EGFR/ErbB). Vascular Endothelial Growth Factor plays an essential role in regulating SMS cell proliferation and differentiation, and induces apoptosis, survival, or cell proliferation by activating various types of signal transduction pathways. The vascular endothelial growth factor includes, for example, vascular endothelial growth factors of humans and non-human animals (e.g., mice).

Vascular endothelial growth factor is included in a concentration that allows appropriate culturing of pluripotent stem cells. Vascular endothelial growth factor may be included in the fifth medium at a concentration of, for example, 5 to 95 ng/ml, 10 to 90 ng/ml, 15 to 85 ng/ml, 20 to 80 ng/ml, 25 to 75 ng/ml, 30 to 70 ng/ml, 35 to 65 ng/ml, 40 to 60 ng/ml, 45 to 55 ng/ml or 50 ng/ml.

Basic Fibroblast Growth Factor (bFGF) is a protein belonging to the FGF family that functions as a mitogen, angiogenic factor, osteogenic factor, and neurotrophic factor, and is also involved in cell proliferation and differentiation. Basic Fibroblast Growth Factor, also called FGF2, mainly activates receptor proteins including FGFR1b, FGFR1c, FGFR2c, FGFR3c, and FGFR4c, and particularly strongly activates FGFR1c and FGFR3c.

Basic fibroblast growth factor is included in the fifth medium at a concentration that allows appropriate culturing of pluripotent stem cells together with other components. Basic fibroblast growth factor may be included in the fifth medium at a concentration of, for example, 1 to 40 ng/ml, 5 to 35 ng/ml, 10 to 30 ng/ml, 15 to 25 ng/ml or 20 ng/ml.

The sixth medium may not include a transforming growth factor beta (TGFβ) signaling inhibitor. When the sixth medium includes a TGFβ signaling inhibitor, differentiation may occur before sufficient cell proliferation takes place.

The sixth medium is a growth medium. Like the fifth medium, it includes at least growth factor such as vascular endothelial growth factor (VEGF) and basic fibroblast growth factor (bFGF). The sixth medium may further include other growth factors for culturing pluripotent stem cells.

The provisions regarding vascular endothelial growth factor (VEGF) and basic fibroblast growth factor (bFGF) in the sixth medium shall apply to those regarding vascular endothelial growth factor (VEGF) and basic fibroblast growth factor (bFGF) in the fifth medium.

The sixth medium may include a transforming growth factor beta (TGFβ) signaling inhibitor.

Transforming growth factor beta (TGFβ) signaling inhibitor refers to a substance that inhibits TGFβ signaling. TGFβ is a substance that regulates various physiological processes in the body, such as cell proliferation, differentiation, apoptosis, migration, production of extracellular matrix (ECM), angiogenesis, and development.

The TGFβ signaling inhibitor may be used without limitation as long as it is a substance that can inhibit TGFβ signaling, and may be, for example, an activin receptor-like kinase (ALK) receptor inhibitor.

The activin receptor-like kinase receptor inhibitor may be, but is not limited to, an ALK5, ALK4, and ALK7 receptor inhibitor. For example, the ALK receptor inhibitor may be SB431542. SB431542 may be represented by the following chemical name: 4-[4-(2H-1,3-benzodioxol-5-yl)-5-(pyridin-2-yl)-1H-imidazol-2-yl]benzamide.

A transforming growth factor beta (TGFβ) signaling inhibitor is included at a concentration sufficient for culturing pluripotent stem cells together with other components. The transforming growth factor beta (TGFβ) signaling inhibitor may be included at a concentration of, for example, 1 to 20 µM, 5 to 15 µM, or 10 µM.

The culture medium obtained through (S1) step of culturing includes hematopoietic stem cells (HSCs). The culture medium may further include hematopoietic progenitor cells (HPCs) and megakaryocyte progenitor cells (MK-Ps).

(S2) of the red blood cell production method of the present invention is a step of obtaining a CD41⁻/CD34⁻ cell population among the cells included in the culture medium of (S1).

Any conventional technique known in the art that is used to select cells based on marker expression in a cell population may be used without limitation. For example, an antibody-based selection method or a sorter machine may be used, and the antibody-based selection method may be specifically a method using microbeads.

After selecting CD41⁻ cells in step (S2), CD34⁻ cells may be selected, or cells may be selected in the reverse order.

(S3) of the red blood cell production method of the present invention is a step of maturing the cell population obtained in (S2) in a first medium including erythropoietin (EPO).

The first medium is a medium including at least one growth factor, erythropoietin, in the basal medium.

The maturation step of (S3) may be performed for 4 to 14 days, and through this process, the red blood cell production method of the present invention may produce red blood cells in a short period of time.

The red blood cell production method of the present invention may further include proliferating the cell population obtained in (S2) in a second medium and differentiating the cell population in a third medium prior to (S3).

The second medium may further include at least erythropoietin, stem cell factor and interleukin-3 in addition to the basal medium. The above proliferation phase may be performed for 6 to 10 days, and is preferably performed for about 8 days.

The third medium may further include at least erythropoietin and stem cell factor in the basal medium. The above differentiation step may be performed for 3 to 7 days, and is preferably performed for about 5 days.

The red blood cell production method of the present invention selects cells not expressing specific markers (CD34, CD41a) and uses them for producing red blood cells, to thus increase not only the differentiation efficiency into red blood cells but also the cell proliferation ability, thereby efficiently producing a large quantity of red blood cells.

Efficiency of differentiation into erythrocytes may be confirmed by the production of a large number of cells expressing CD235 (glycophorin A) as an erythrocyte surface marker, when cells not expressing CD34 and CD41a are subsequently cultured.

The method for producing platelets according to the present invention may include the steps of (S1) culturing pluripotent stem cells to obtain a culture medium including hematopoietic stem cells; (S2) sorting a population of CD41⁻/CD34⁻ cells and a population of CD41a⁺ or CD34⁺ cells from the culture medium; and (S4) differentiating the sorted population of CD41a⁺ or CD34⁺ cells into megakaryocytes in a seventh medium.

The above pluripotent stem cells may be within the range described above.

The above (S1) and (S2) may be within the above-described range. (S2) of the platelet producing method of the present invention may be a cell population excluding the CD41⁻/CD34⁻ cell population selected in (S2) of the above-described red blood cell production method.

Step (S4) is the step of differentiating the CD41a⁺ or CD34⁺ cell population sorted in step (S2) into megakaryocytes in the seventh medium.

The seventh medium serves to differentiate the cell population into megakaryocytes. The seventh medium may contain cytokines. The seventh medium may include at least one of thrombopoietin (TPO), stem cell factor (SCF), interleukin-3 (IL-3), and interleukin-6 (IL-6).

Thrombopoietin is a major growth factor that regulates megakaryocyte and platelet hematopoiesis and is mainly synthesized and secreted by hepatocytes.

Thrombopoietin may be included in the seventh medium at a concentration that allows culturing of the suspension cell population together with other components and does not have to be included at a specific concentration. Thrombopoietin may be included at a concentration of, for example, 1 to 50 ng/mL, 5 to 45 ng/mL, 10 to 40 ng/mL, 15 to 35 ng/mL, 20 to 30 ng/mL, or 25 ng/mL

Stem cell factor is a stromal cell-derived cytokine synthesized by fibroblasts and other cell types. The stem cell factor may be included in the seventh medium at a concentration that allows culturing of the suspension cell population together with other components and does not have to be included at a specific concentration. Thrombopoietin may be included at a concentration of, for example, 1 to 50 ng/ml, 5 to 45 ng/ml, 10 to 40 ng/ml, 15 to 35 ng/ml, 20 to 30 ng/ml, or 25 ng/ml.

Interleukin-3 may be included in the seventh medium at a concentration that allows culturing of a population of floating cells together with other components, and does not have to be included at a specific concentration. Interleukin-3 may be included at a concentration of, for example, 1 to 20 ng/ml, 5 to 15 ng/ml, or 10 ng/ml.

Interleukin-6 may be included in the seventh medium at a concentration that allows culturing of a population of floating cells together with other components, and does not have to be included at a specific concentration. Interleukin-6 may be included at a concentration of, for example, 1 to 20 ng/ml, 5 to 15 ng/ml, or 10 ng/ml.

The seventh medium is not limited to a specific medium. The seventh medium may include Iscove's Modified Dulbecco's Medium (IMDM) as a basal medium. The IMDM medium may further include an antioxidant (e.g., AA2P, etc.) and/or B-27.

The method for producing platelets of the present invention may further include maturing megakaryocytes by culturing them in an eighth medium including thrombopoietin.

In the eighth medium, thrombopoietin may be included in a concentration of, for example, but not limited to, 50 to 150 ng/mL, 60 to 140 ng/mL, 70 to 130 ng/mL, 80 to 120 ng/mL, 90 to 110 ng/mL or 100 ng/mL

The eighth medium may include additional factors necessary for the maturation of megakaryocytes in addition to thrombopoietin.

The eighth medium is not limited to a specific medium. The eighth medium may include Iscove's Modified Dulbecco's Medium (IMDM) as a basal medium. The IMDM medium may further include an antioxidant (e.g., Ascorbic Acid 2-Phosphate, AA2P, etc.) and/or B-27.

In this step, mature megakaryocytes or a culture medium including megakaryocytes and/or mature megakaryocytes may be obtained.

In the method for producing platelets of the present invention, platelets may be separated and/or purified from the culture medium after differentiating megakaryocytes further, or platelets may be separated and/or purified from the culture medium without further differentiation.

Isolation and/or purification of platelets may be performed by any known separation and/or purification method, for example, by centrifuging the culture medium or passing the culture medium through a column.

When the culture medium is centrifuged, megakaryocytes may be separated as a precipitate while platelets may be separated as a suspension.

The present invention relates to a method for producing red blood cells and platelets which may implement the above-described red blood cell production method and the platelet producing method together in one routine.

The method for producing red blood cells and platelets of the present invention may conduct the steps of (S3) maturing a population of sorted CD41⁻/CD34⁻ cells in a first medium including erythropoietin and (S4) differentiating a population of sorted CD41a⁺ or CD34⁺ cells into megakaryocytes in a seventh medium simultaneously or sequentially.

When performed sequentially, (S3) may be performed after (S4), or conversely, (S4) may be performed after (S3).

The method for producing red blood cells and platelets of the present invention can produce red blood cells and platelets together in a single process, to thus economically use the cultured cells, and may increase the production efficiency by selecting cells suited to the characteristics of red blood cells and platelets and differentiating them respectively.

The present invention will be described in more detail with the following examples.

### Example 1. Preparation and analysis of red blood cells

### 1. Preparative Example 1 of red blood cell

### 1-1 Culturing of human induced pluripotent stem cells (hiPSC)

Human induced pluripotent stem cells were cultured as follows to obtain a population of cells capable of differentiating into erythrocytes.

First, human induced pluripotent stem cells were seeded at a density of 4000 cells/cm² in a vitronectin-coated T75 flask containing mTeSR plus culture medium together with 10 µg/ml of ROCK inhibitor (Y-27632) and cultured at 37°C and 5% CO₂. After 24 hours of culture, Y-27632 was removed, the cells were washed once with PBS, and the mTeSR plus culture medium was replaced with fresh medium. The culture medium was replaced once a day and cultured for 3 days to secure cells for differentiation.

Afterwards, human induced pluripotent stem cells were cultured in RPMI1640 basal medium including 1% P/S, 1% GlutaMAX, 300 uM AA2P and 2% B-27, which is supplemented with 6 µM CHIR99021 as a GSK3 (glycogen synthase kinase 3) inhibitor, for 2 days under conditions of 37°C and 5% CO₂, while replacing the culture medium once a day (differentiation initiation stage).

To differentiate human induced pluripotent stem cells into cells that can differentiate into megakaryocytes, the medium composition was changed to RPMI1640 basal medium including 1% P/S, 1% GlutaMAX, 300 uM AA2P and 2% B-27, which is supplemented with 50 ng/ml VEGF and 20 ng/ml bFGF. While the culture medium was replaced once a day, cells were cultured in an incubator at 37°C, 5% CO₂ for 3 days. The culturing was performed until the cell confluency reached approximately 90%.

Afterwards, 10 µM SB431542 together with VEGF and bFGF were added to RPMI1640 basal medium including 1% P/S, 1% GlutaMAX, 300 uM AA2P and 2% B-27, and the culturing was performed for additional 4 days while replacing the culture medium once every 2 days.

A floating cell population was obtained by culturing, and CD41a⁻/CD34⁻ cells and CD41a⁻/CD34⁺ cells were isolated from the floating cell population using CD41a and CD34 microbeads.

### 1-2 Culturing the selected cell population to induce differentiation into erythrocytes

CD41a⁻/CD34⁻ cells and CD41a⁻/CD34⁺ cells were selected from the floating cell population obtained in the above "1-1 Culture of human induced pluripotent stem cells (hiPSC)" and cultured.

CD41a⁻/CD34⁻ cells and CD41a⁻/CD34⁺ cells were seeded at a density of 1×10⁵ cells/ml in IMDM basal medium including 300 uM AA2P and 2% B-27, respectively, and cultured (proliferated) for 8 days at 37°C, 5% CO₂ in the medium supplemented with 6 U/ml erythropoietin (EPO), 100 ng/ml SCF, and 10 ng/ml IL-3.

Thereafter, the medium composition was changed to a medium including 6 U/ml EPO and 100 ng/ml SCF supplemented in IMDM basal medium including 300 uM AA2P and 2% B-27, and the cells were further cultured (differentiated) for 5 days (this proliferation and differentiation process is referred to as the differentiation method (Diff) in the present invention), and the medium composition was changed to a medium including 6 U/ml EPO supplemented in IMDM basal medium including 300 uM AA2P and 2% B-27, and the cells were further cultured (matured) for 8 days to obtain a cell culture.

Since red blood cells become red due to hemoglobin when differentiated, the color of the cell pellet was checked to confirm this color.

The culture media of the two cell populations obtained above were placed in a 15 ml conical tube and centrifuged at 400×g for 5 minutes at room temperature (RT). The supernatant was removed, and the pellet was resuspended in 1 ml 1×PBS, transferred to a 1.7 ml tube, and centrifuged at 400×g for 5 minutes at room temperature (RT). The tube was laid flat so that the pellets could be easily seen, and the pellets were photographed.

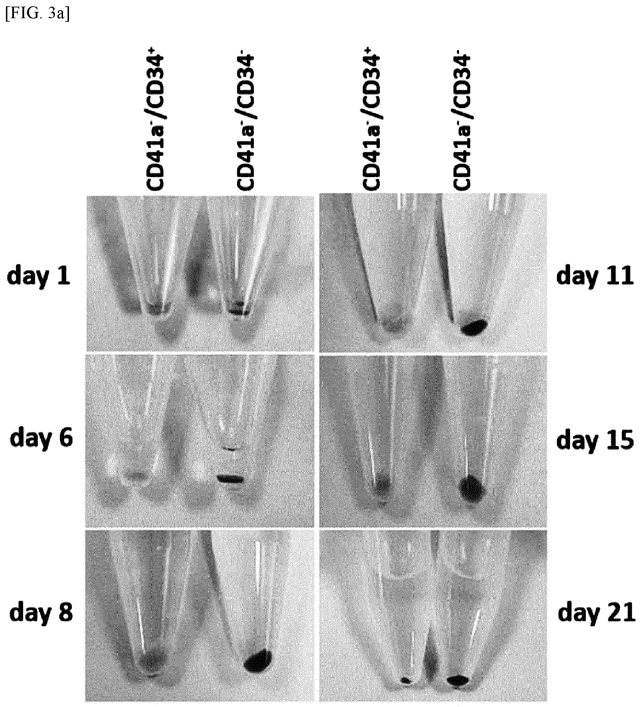

As a result, the CD41a⁻/CD34⁻ cell pellet was red, whereas the CD41a⁻/CD34⁺ cells were not red, confirming that the material obtained by culturing CD41a⁻/CD34⁻ cells had differentiated into erythrocytes (FIG. 3A).

### 2. Preparative Example 2 of red blood cell

### 2-1 Culturing of human induced pluripotent stem cells (hiPSC)

Human induced pluripotent stem cells were cultured using the same method as in the above "1-1 Culture of human induced pluripotent stem cells (hiPSC)," and CD41a⁻/CD34⁻ cells and CD41a⁻/CD34⁺ cells were selected.

### 2-2 Culturing the selected cell population to induce differentiation into erythrocytes

CD41a⁻/CD34⁻ cells and CD41a⁻/CD34⁺ cells were selected from the floating cell population obtained in the above "2-1 Culture of human induced pluripotent stem cells (hiPSC)" and cultured.

CD41a⁻/CD34⁻ cells and CD41a⁻/CD34⁺ cells were seeded at a density of 1×10⁵ cells/ml in IMDM basal medium including 300 uM AA2P and 2% B-27, respectively, and 6 U/ml EPO was added to the medium.
The cells were cultured (matured) for 14 days at 37°C and 5% CO ₂ to obtain a cell culture.

The culture media of the two obtained cell populations were placed in a 15 ml conical tube and centrifuged at 400×g for 5 minutes at room temperature (RT). The supernatant was removed, and the pellet was resuspended in 1 ml 1×PBS, transferred to a 1.7 ml tube, and centrifuged at 400×g for 5 minutes at room temperature (RT). The tube was laid down so that the pellets could be easily seen, and the pellets were photographed.

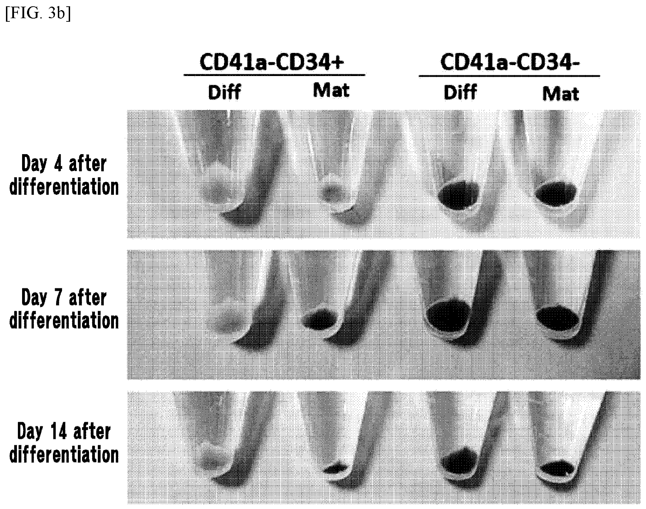

As a result, the CD41a⁻/CD34⁻ cell pellet was red, whereas the CD41a⁻/CD34⁺ cells were not red, confirming that the material obtained by culturing CD41a⁻/CD34⁻ cells had differentiated into erythrocytes (FIG. 3B).

### 3. Confirmation of whether the red blood cells are normal

### 3-1 Blood cell staining (Wright-Giemsa staining)

To confirm whether the material obtained by culturing CD41a⁻/CD34⁻ cells and CD41a⁻/CD34⁺ cells in 1-2 and 2-2 above had differentiated into erythrocytes, Wright-Giemsa staining was performed.

Wright-Giemsa stain is a blood smear staining technique in which red blood cells are stained pink, platelets are stained pale pink, the cytoplasm of lymphocytes is stained light blue, and the cytoplasm of monocytes is stained blue.

The experimental method is as follows. Cells were centrifuged at 400×g for 5 minutes at room temperature (RT), resuspended in 100 µl of 4% PFA (paraformaldehyde), and fixed for 15 minutes at 4°C. After centrifugation at 400×g for 5 minutes at 4°C, the cells were resuspended in 30 µl of 1x PBS, and 5 µl of the solution was placed on a slide glass and dried on a 56°C heat block. After the slides were completely cooled, the cells were reacted with 0.1% Triton X-100 for 10 minutes at room temperature. After washing with 1x PBS, they were stained with giemsa solution for 10 minutes. The cells were washed with distilled water (DW) and observed under a microscope (observation magnification 400×).

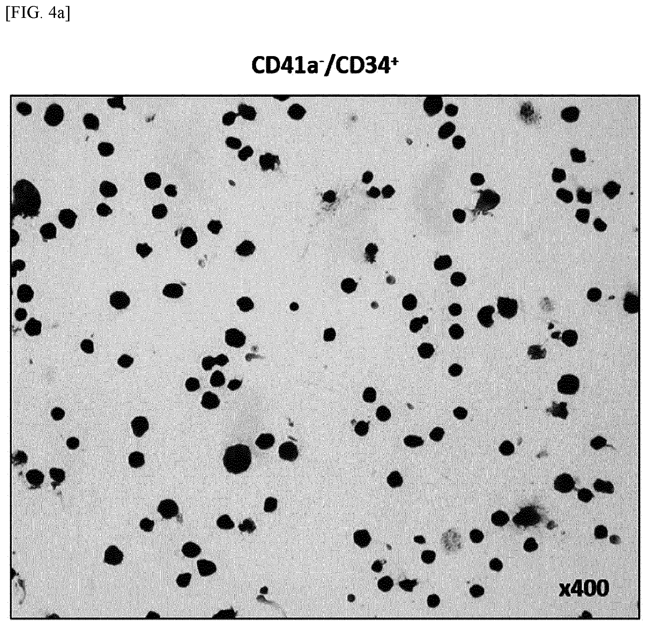

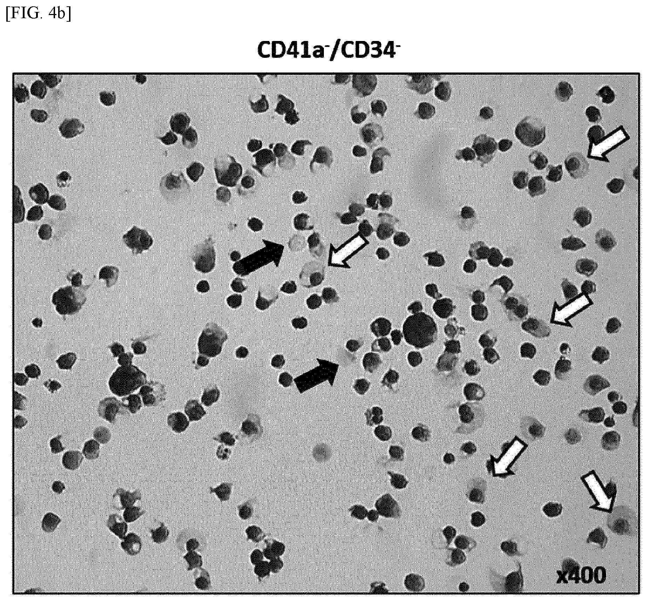

As a result, the material obtained by culturing CD41a⁻/CD34⁻ cells was observed to have orthochromatic normoblasts (red blood cells with nuclei: white arrows) and reticulocytes (red blood cells without nuclei: black arrows) with nuclei shifted to one side (FIG. 4).

During the red blood cell differentiation process, the nucleus shifts to one side before enucleation and, after enucleation, cells with only cytoplasm are created. By observing all of these cells in the material obtained by culturing CD41a⁻/CD34⁻ cells, it was confirmed that this material was a red blood cell.

### 3-2 Immunostaining

To confirm whether the materials obtained in 1-2 and 2-2 above were red blood cells, the expression of CD235, a red blood cell surface marker, and the presence of nuclei were investigated by immunostaining.

Cells were centrifuged at 400×g for 5 minutes and resuspended with 200 µl of 1% BSA. CD235-PE antibody was added and reacted at 4°C for 30 minutes. The reacted cells were centrifuged at 400×g for 5 minutes at 4°C, the supernatant was removed, and 500 µl of 1×PBS was added and washed. The washed cells were resuspended in 30 µl 1×PBS, by adding 1µl of Hoechst 33342 and observation under a fluorescence microscope.

To confirm CD235 expression, CD235-PE antibody was reacted with each cell, and to confirm the presence of nuclei, Hoechst 33342 was reacted with each cell.

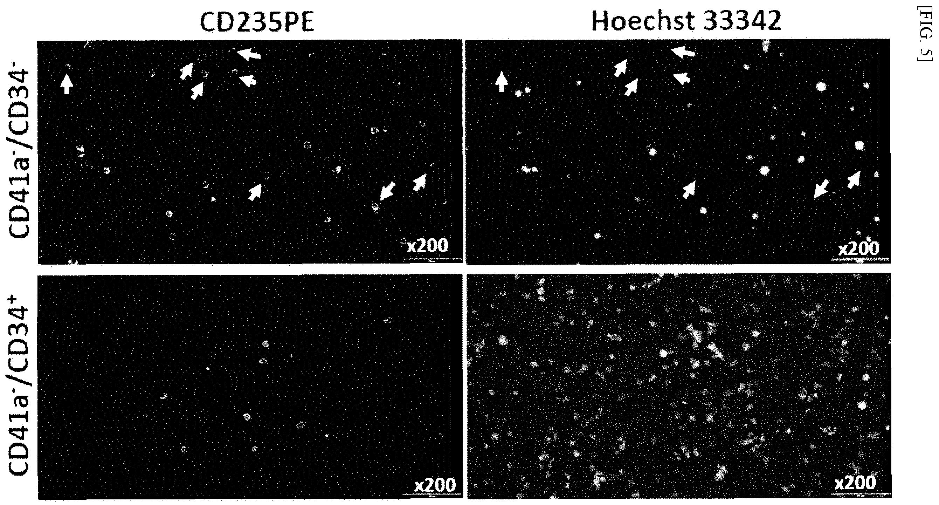

As a result, as shown in FIG. 5, in the material obtained by culturing CD41a⁻/CD34⁻ cells, cells (white arrows) were observed in which only CD235 was stained but the nucleus was not stained. They may be reticulocytes that express CD235 during the erythroid differentiation process and do not have a nucleus. On the other hand, in the material obtained by culturing CD41a⁻/CD34⁺ cells, cells expressing CD235 were hardly found, while cells in which CD235 was stained but the nucleus was not stained were neither found.

### 3-3 FACS Analysis

FACS analysis was performed to confirm whether the materials obtained in the above 1-2 and 2-2 express CD71 and CD235, which are red blood cell-specific markers. FACS analysis was performed using a BD Lyric instrument, and anti-CD71-APC and anti-CD235-PE antibodies were used. The antibodies were diluted 1:20 in PBS (FACS buffer) containing 1% BSA, and reacted for 30 minutes to confirm surface marker expression.

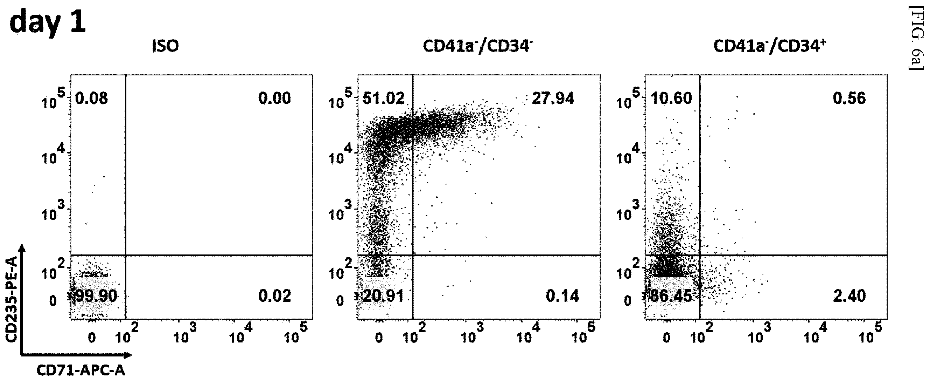

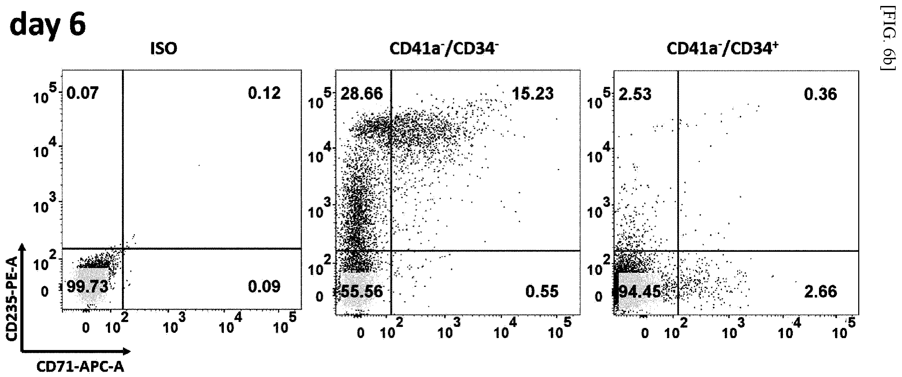

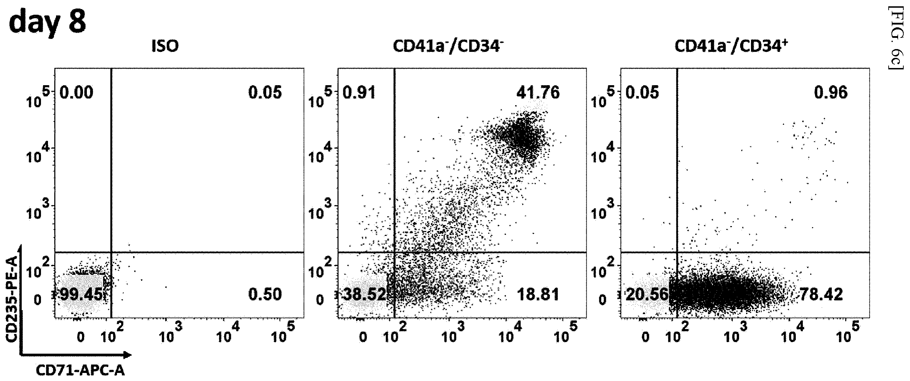

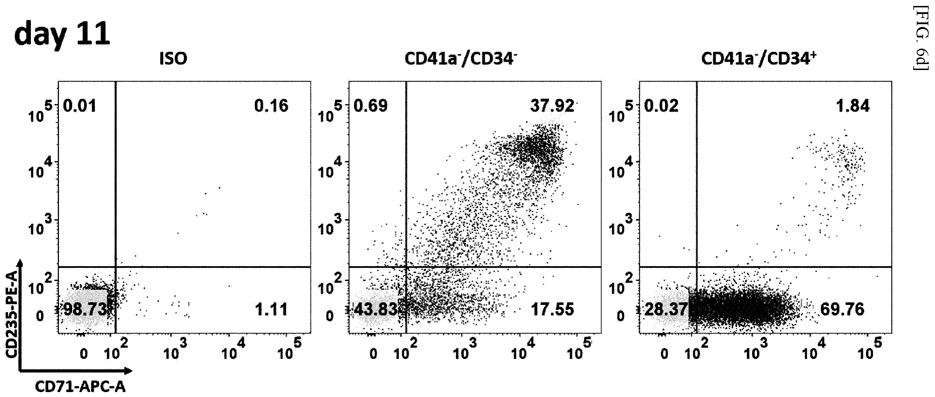

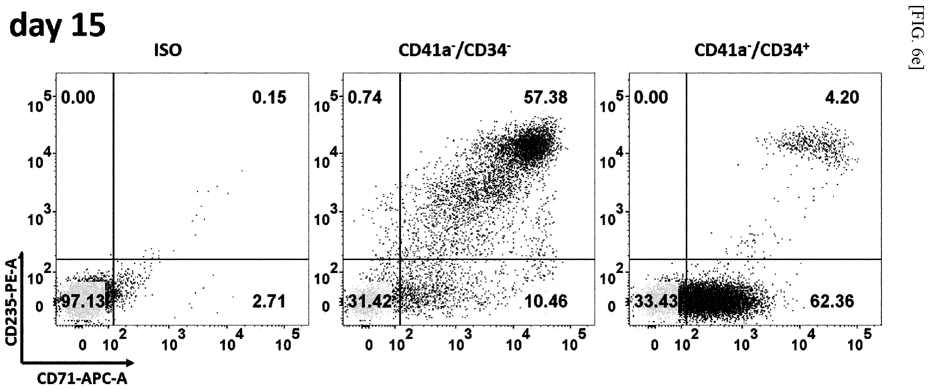

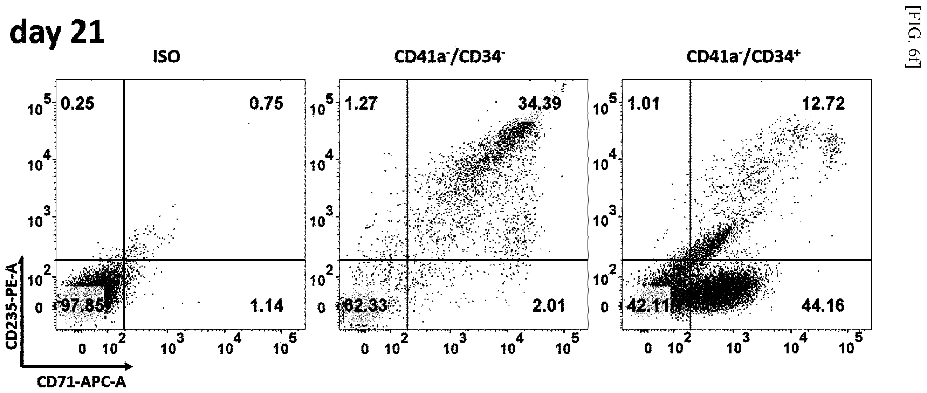

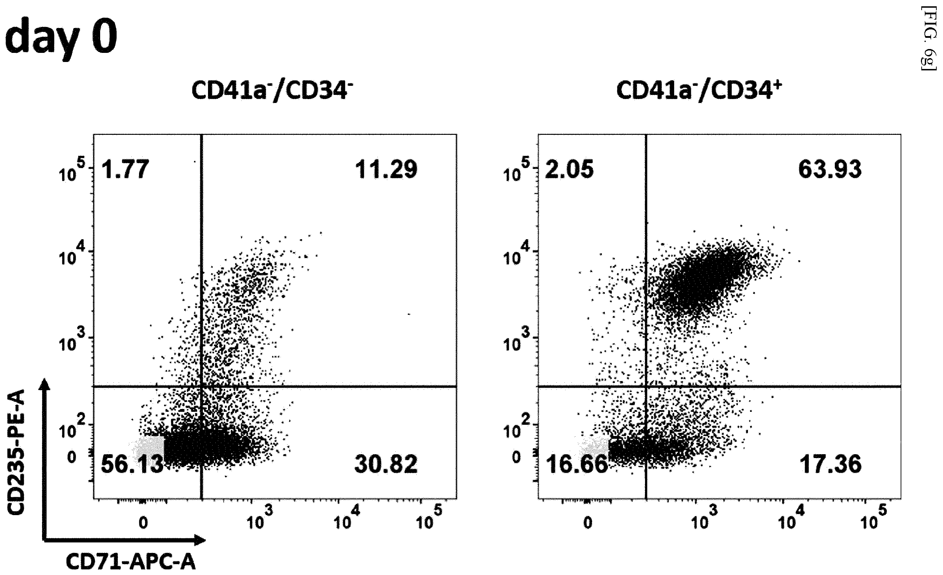

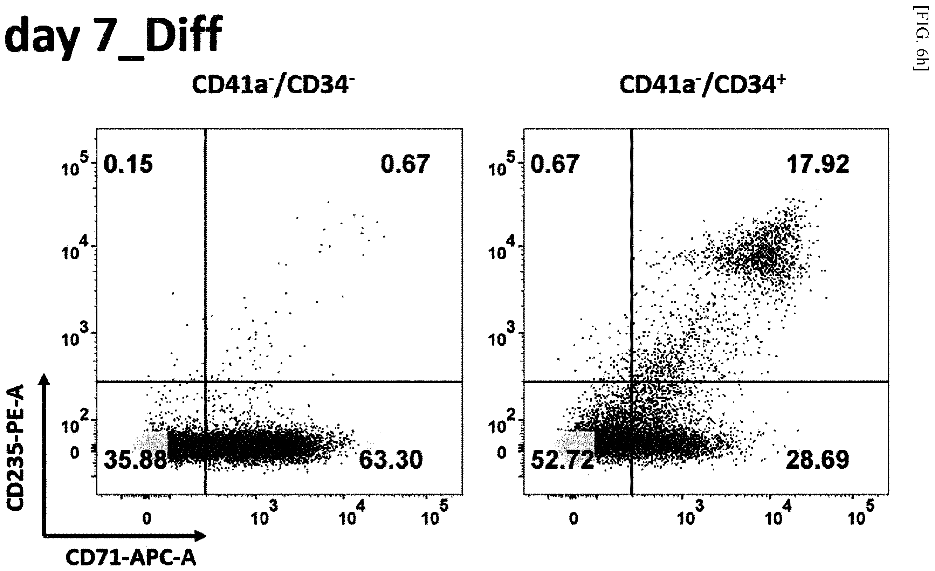

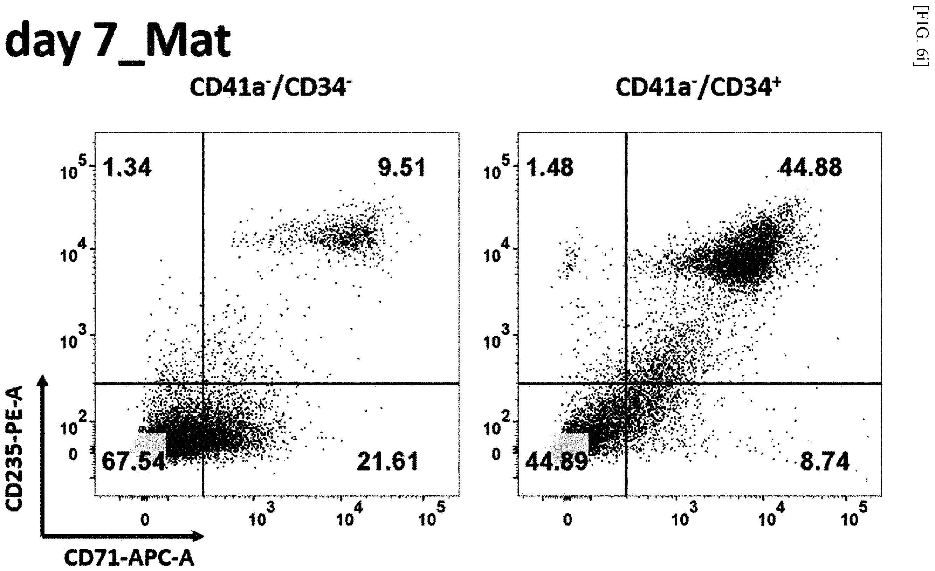

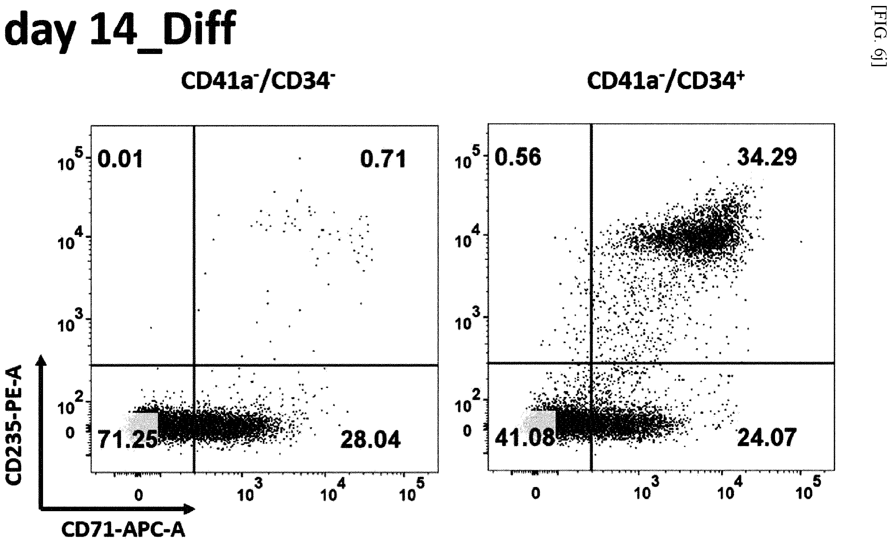

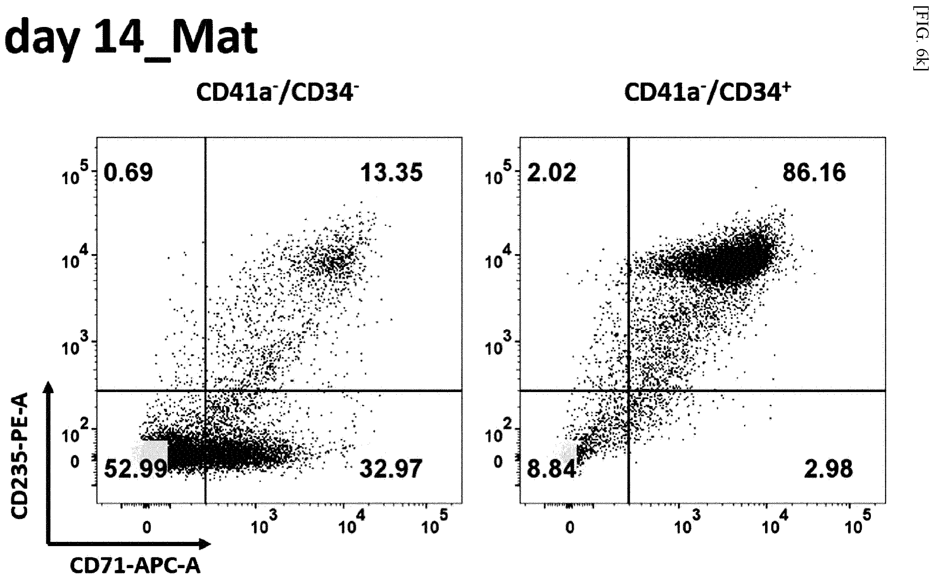

As a result, the material obtained from CD41a⁻/CD34⁻ cells was positive for the expression of erythrocyte-specific markers (CD71, CD235) (FIG. 6). This confirmed that the material was an erythrocyte.

Meanwhile, it was confirmed that the material obtained from CD41a⁻/CD34⁺ cells was not an erythrocyte, as the expression of CD71 and CD235 was very low (FIG. 6).

### 3-4 Cell counting

To determine the cell proliferation capacity of the CD41a⁻/CD34⁻ cell and CD41a⁻/CD34⁺ cell populations, the cell proliferation rate was measured by cell count during the induction of differentiation into erythrocytes in the above 2.

20 µl of cells and 20 µl of 0.4% trypan blue were mixed, and 10 µl of the mixture was counted after 1 minute using a Countess 3 cell counter machine.

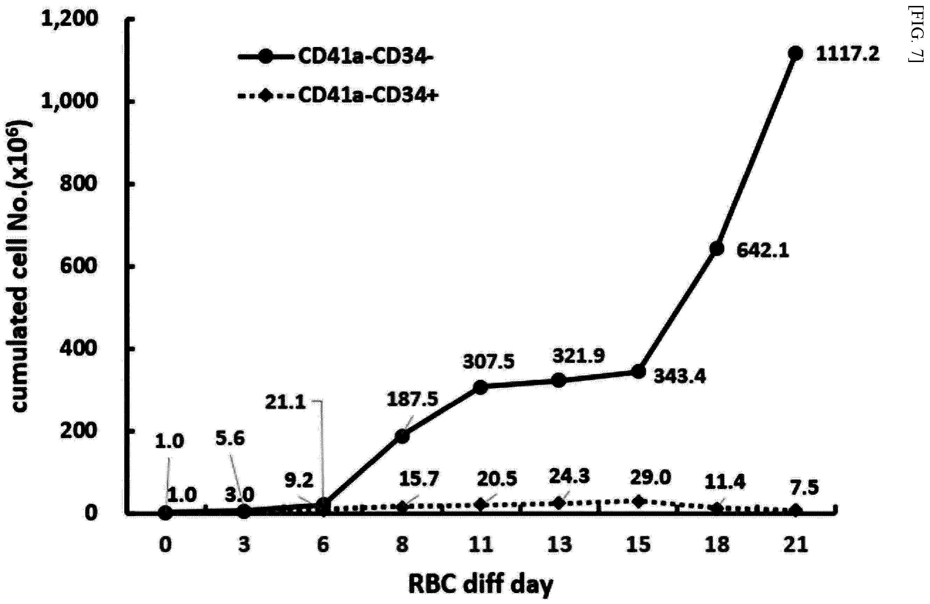

As a result, the CD41a⁻/CD34⁻ cell population showed a higher cell proliferation rate than the CD41a⁻/CD34⁺ cell population, confirming that a larger amount of red blood cells could be produced when red blood cells were produced using CD41a⁻/CD34⁻ cells (FIG. 7).

### 3-5 Hemoglobin ELISA

ELISA was performed to determine the amount of hemoglobin in the CD41a⁻/CD34⁻ cell and CD41a⁻/CD34⁺ cell populations.

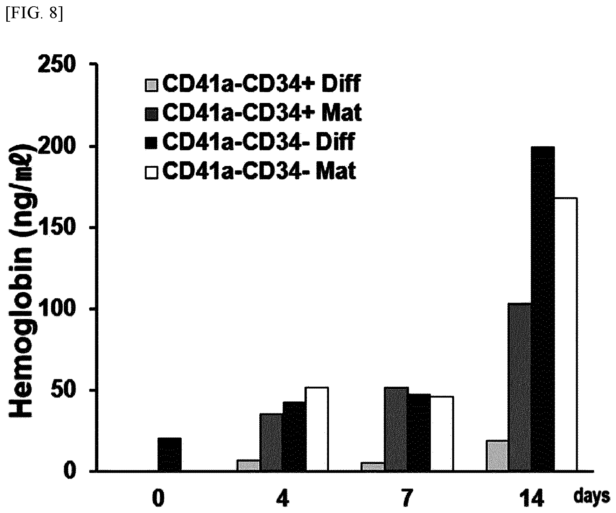

1x10⁶ cells were lysed in 30 µl RIPA buffer at 4°C for 30 minutes, centrifuged at 12,000 rpm and 4°C for 30 minutes, and the supernatant was obtained. 25 µl of the supernatant was diluted with 225 µl of dilution buffer, and 100 µl of the solution was loaded per well. The results were obtained by experimenting according to the manufacturer's protocol for Ab157707 (FIG. 8).

### 3-6 Transmission electron microscopy (TEM)

To observe CD41a⁻/CD34⁻ cells, transmission electron microscopy was performed as follows.

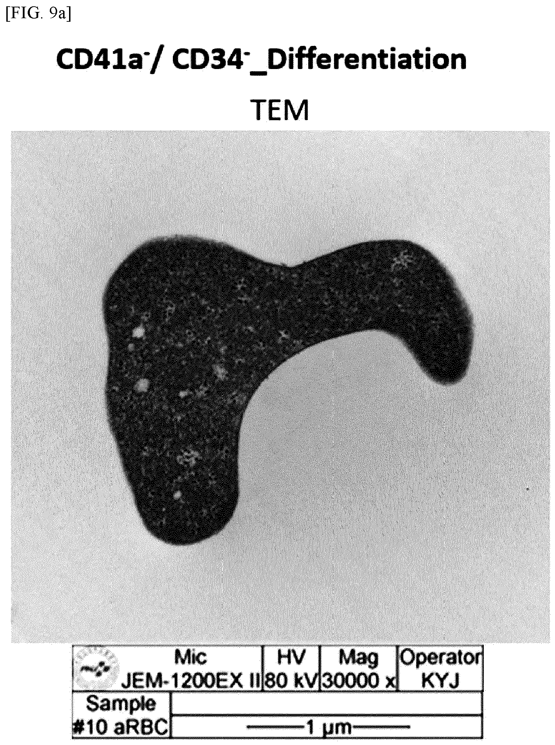

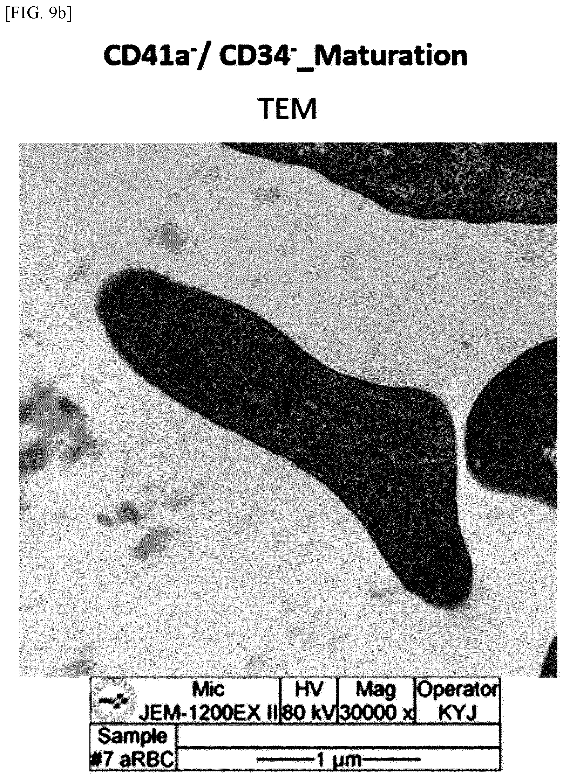

CD41a⁻/CD34⁻ cells were pre-fixed with 2.5% glutaraldehyde (4°C, phosphate buffer, pH 7.2). Then, the cells were post-fixed with 1% osmium tetroxide (OsO4, 4°C, phosphate buffer, pH 7.2). After fixation, the samples were washed with the same buffer and dehydrated with increasing alcohol concentrations (30%, 50%, 70%, 80%, 90%, 95%, 100%, 100%, 100%). The treated samples were embedded and solidified with Epon 812 mixture. After the embedding was completed, the tissues were sliced into 1 µm thick sections, stained with 1% toluidine blue, and a specific area was designated. After trimming the remaining area, ultrathin sections with a thickness of 50-70 nm were obtained using an ultramicrotome (EM UC7, Leica), double stained with uranyl acetate and lead citrate, and observed under a transmission electron microscope (JEM-1200EXII, JEOL) (FIG. 9).

### 3-7 Scanning Electron Microscopy (SEM)

To observe CD41a⁻/CD34⁻ cells, scanning electron microscopy was performed as follows.

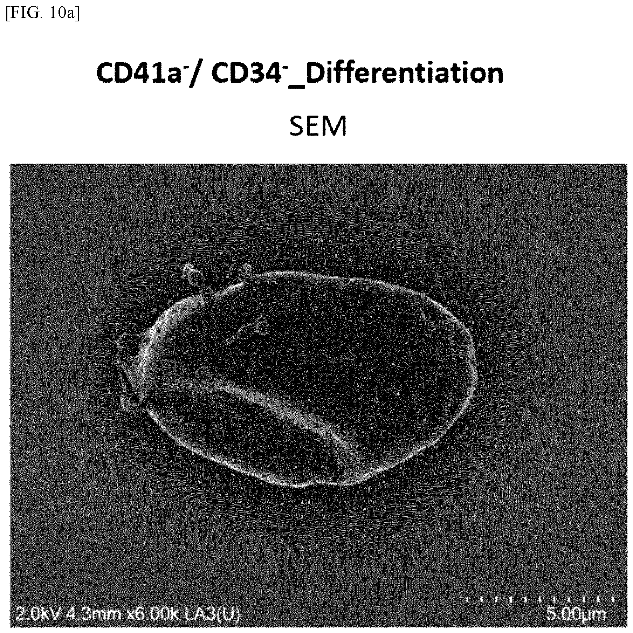

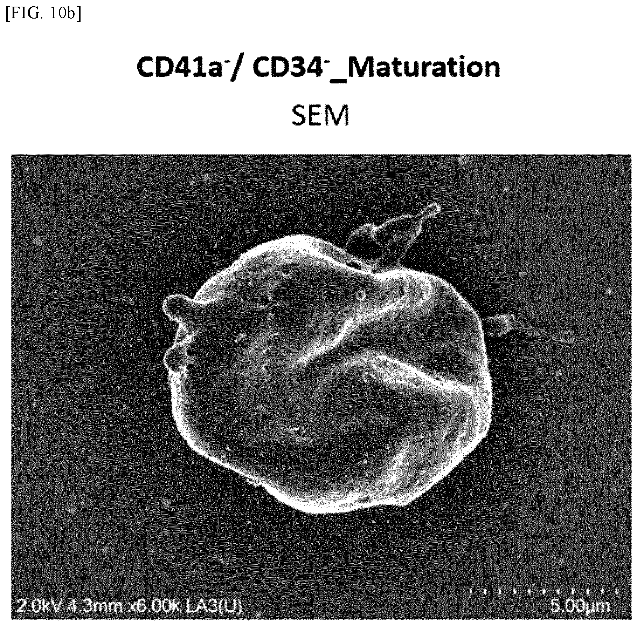

Cells were pre-fixed with 2.5% glutaraldehyde (4°C, phosphate buffer, pH 7.2). Then, the cells were post-fixed with 1% osmium tetroxide (OsO4, 4°C, phosphate buffer, pH 7.2). After fixation, samples were washed with the same buffer and dehydrated with increasing alcohol concentration (30%, 50%, 70%, 80%, 90%, 95%, 100%, 100%, 100%). Ethanol: HMDS (Hexamethyldisilazane) = 3:1, 1:1, 1:3 ratios were used to replace 100% HMDS. Samples were placed on silicon wafers and dried. After Pt coating (SMC12R-Plus (Semian, Korea): 15 mA, 2-3 min), cells were observed using Regulus 8220 (Hitachi, Japan) (FIG. 10).

### Example 2. Platelet production and analysis

### 1. Culturing of human induced pluripotent stem cells (hiPSC)

Human induced pluripotent stem cells were cultured as follows to obtain a cell population capable of differentiating into megakaryocytes.

First, human induced pluripotent stem cells were placed in a T75 flask at a density of 4000/cm2 in mTeSR plus culture medium with 10 µg/ml of ROCK inhibitor (Y-27632), and cultured at 37°C and 5% CO2. After 24 hours of culture, Y-27632 was removed by washing once with PBS and replacing the mTeSR plus culture medium with fresh medium. The culturing was performed for 3 days while replacing the culture medium once a day, thereby securing cells for differentiation.

Afterwards, human induced pluripotent stem cells were cultured in RPMI1640 basal medium including 300 uM AA2P, 2% B-27, which is supplemented with 6 µM CHIR99021 as a GSK3 (glycogen synthase kinase 3) inhibitor, for 2 days under conditions of 37°C and 5% CO₂ (culture medium changed once a day).

To differentiate human induced pluripotent stem cells into cells that can differentiate into megakaryocytes, the medium composition was changed to RPMI1640 basal medium including 300 uM AA2P and 2% B-27, which is supplemented with 50 ng/mL VEGF and 20 ng/mL bFGF, followed by culturing in an incubator with 5% CO₂ at 37°C for 3 days (changing the culture medium once a day). The culturing was performed until the cell confluency reached approximately 70%. Thereafter, 10 µM SB431542 together with VEGF and bFGF were further added to RPMI1640 basal medium including 300 uM AA2P and 2% B-27, followed by additional culturing for a predetermined period of time.

### 1) Floating Cell Preparative Example 1

On the fourth day of additional culture, the ratio of cells expressing specific surface markers (CD34, CD41a, CD45) to the total number of floating cells was investigated through flow cytometry (FACS). For flow cytometry, a BD Lyric instrument was used, and BD's anti-CD34-FITC, anti-CD45-FITC, and anti-CD41a-APC antibodies were used. Through this, the ratio of single or double positive cells of CD34 and CD41a, and CD45 and CD41a, respectively, was analyzed.

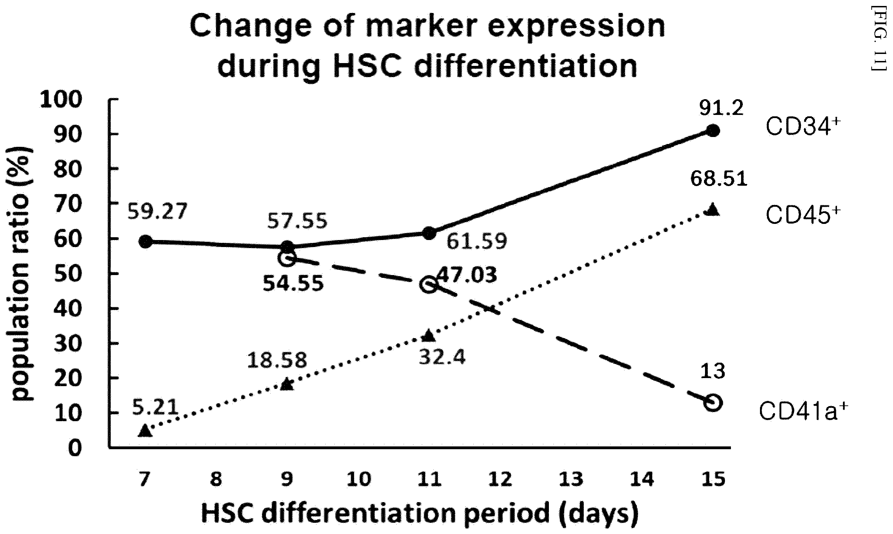

As a result, it was confirmed that the proportion of CD34⁺ cells was 57.55%, the proportion of CD41a⁺ cells was 54.55%, and the proportion of CD45⁺ cells was 18.58% of the total cells. At the time point when the proportion of CD34⁺ cells in the floating cells was 57.55%, the proportion of CD41a⁺ cells was 54.55% and the proportion of CD45⁺ cells was 18.58%, a floating cell population (day 9 in FIG. 11) was obtained from the culture medium.

### 2) Floating Cell Preparative Example 2

On the sixth day of additional culture, the ratio of cells expressing specific surface markers (CD34, CD41a, CD45) to the total number of floating cells was investigated through flow cytometry. As a result, the ratio of CD34⁺ cells was 61.59%, the ratio of CD41a⁺ cells was 47.03%, and the ratio of CD45⁺ cells was 32.4% of the total cells. A population of floating cells (days 11-12 in FIG. 11) was obtained from the culture medium at the time point when the ratio of CD34⁺ cells was 61.59%, the ratio of CD41a⁺ cells was 47.03%, and the ratio of CD45⁺ cells was 32.4% in the floating cells.

### 3) Floating Cell Preparative Example 3

On the eighth day of additional culture, the ratio of cells expressing specific surface markers (CD34, CD41a, CD45) to the total number of floating cells was investigated through flow cytometry. As a result, the ratio of CD34⁺ cells was 52.05%, the ratio of CD41a⁺ cells was 18.88%, and the ratio of CD45⁺ cells was 45.56% of the total cells. At the time point when the ratio of CD34⁺ cells in the floating cells was 52.05%, the ratio of CD41a⁺ cells was 18.88% and the ratio of CD45⁺ cells was 45.56%, the floating cell population (day 13 in FIG. 11) was obtained from the culture medium.

### 4) Floating Cell Preparative Example 4

On the second day of additional culture, the ratio of cells expressing specific surface markers (CD34, CD45) to the total number of floating cells was investigated through flow cytometry. As a result, the ratio of CD34⁺ cells was 59.27% of the total cells, and the ratio of CD45⁺ cells was 5.21%. At the point when the ratio of CD34⁺ cells in the floating cells was 59.27% and the ratio of CD45⁺ cells was 5.21 %, the floating cell population (day 7 in FIG. 11) was obtained from the culture medium.

### 5) Floating Cell Comparative Preparative Example 1

On the tenth day of additional culture, the ratio of cells expressing specific surface markers (CD34, CD41a, CD45) to the total number of floating cells was investigated through flow cytometry. As a result, it was confirmed that the ratio of CD34⁺ cells was 91.2%, the ratio of CD41a⁺ cells was 13%, and the ratio of CD45⁺ cells was 68.51% of the total cells. At the time point when the ratio of CD34⁺ cells in the floating cells was 91.2%, the ratio of CD41a⁺ cells was 13% and the ratio of CD45⁺ cells was 68.51%, the floating cell population (day 15 in FIG. 11) was obtained from the culture medium.

### 2. Culturing the floating cell population and inducing differentiation into megakaryocytes

For differentiation and maturation into megakaryocytes, the floating cell population obtained in the above "1. Culture of human induced pluripotent stem cells (hiPSC)" was cultured.

In order to differentiate into megakaryocytes from the floating cell population obtained in the above "1. Culture of human induced pluripotent stem cells (hiPSC)," each of the floating cell populations of Floating Cell Preparative Examples 1 to 4 and comparative Preparative Example 1 was placed at a density of 1×10⁵ cells/ml in IMDM basal medium including 300 uM AA2P and 2% B-27, and then, 25 ng/ml TPO, 25 ng/ml SCF, 10 ng/ml IL-3 and 10 ng/ml IL-6 were added to the medium, followed by culturing at 37°C, 5% CO₂ for 3 days.

Afterwards, the medium composition was changed to IMDM basal medium including 300uM AA2P and 2% B-27, which is supplemented with 100 ng/ml TPO, and the cells were cultured for an additional 5 days to perform the maturation (Megakaryocyte maturation) step, thereby obtaining a cell culture medium.

### 3. Confirmation of platelet production capacity of megakaryocytes

Platelets were obtained from the culture media of Floating Cell Preparative Examples 1 and 3 and comparative Preparative Example 1 obtained in the above "2. Induction of differentiation of the floating cell population into megakaryocytes." Specifically, the cell culture media of Floating Cell Preparative Examples 1 and 3 and comparative Preparative Example 1 obtained in the above "2. Induction of differentiation of the floating cell population into megakaryocytes" were all collected and centrifuged at 300×g for 3 minutes. The precipitate was separated as megakaryocytes and the supernatant was separated as platelets, followed by analysis on platelets for these separated products.

Through experiments (1) and (2) below, it was confirmed that the final material obtained by the above-described method was platelets with normal activity.

### (1) Identification of platelet-specific markers through FACS analysis

The cell culture media of Floating Cell Preparative Examples 1 and 3 and comparative Preparative Example 1 were centrifuged to remove precipitated megakaryocytes, and the suspended platelets were centrifuged again at 2000×g for 10 minutes. The platelets precipitated through centrifugation were subjected to FACS analysis to confirm the expression of surface markers. The FACS analysis was performed using a BD Lyric instrument, and BD's anti-CD41a-FITC, anti-CD61-PE and anti-CD42b-APC antibodies were used. The antibodies were diluted 1:20 in PBS (FACS buffer) containing 1% BSA, and reacted for 30 minutes to confirm surface marker expression.

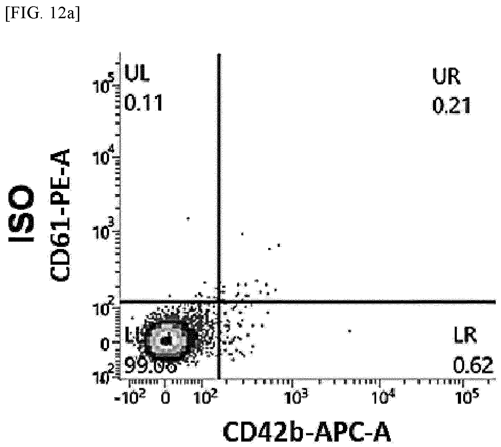

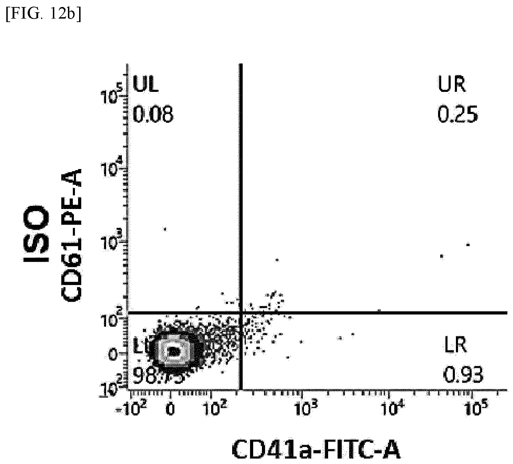

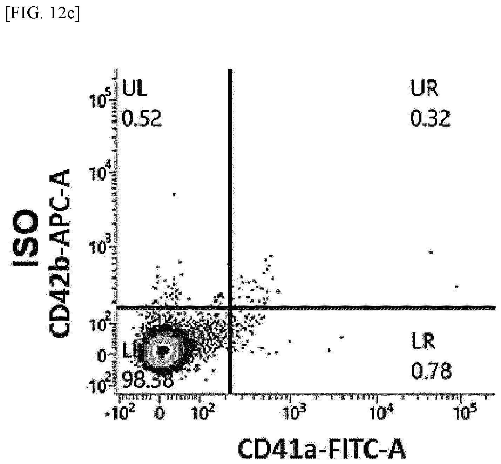

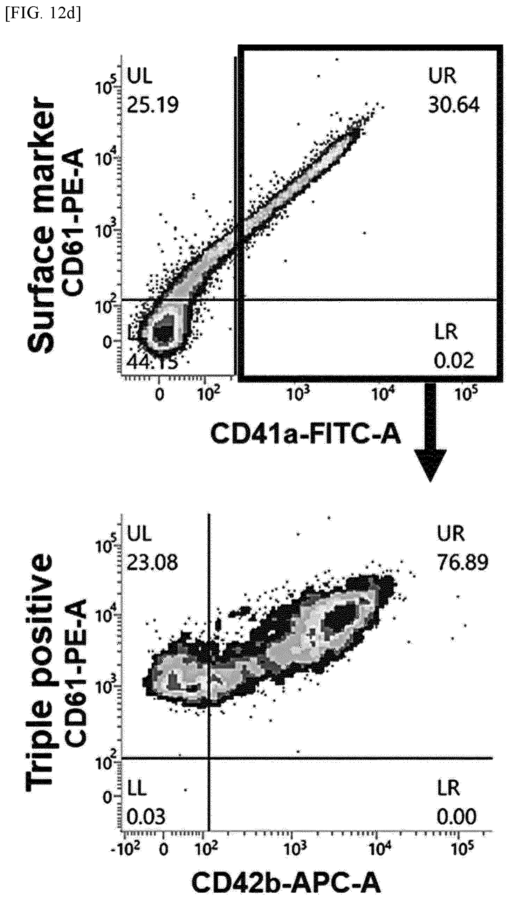

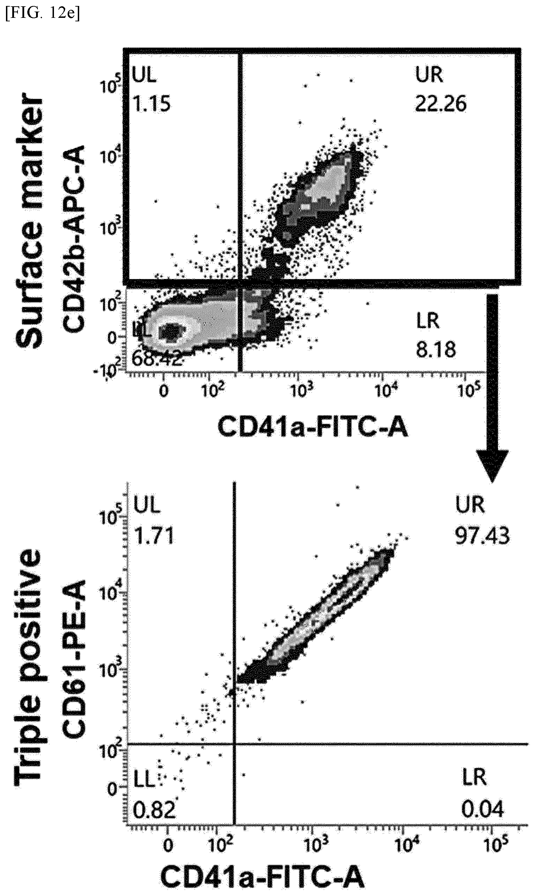

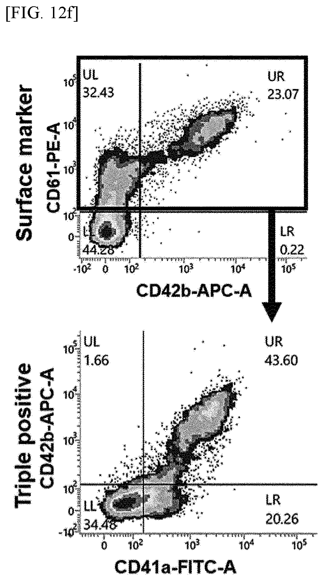

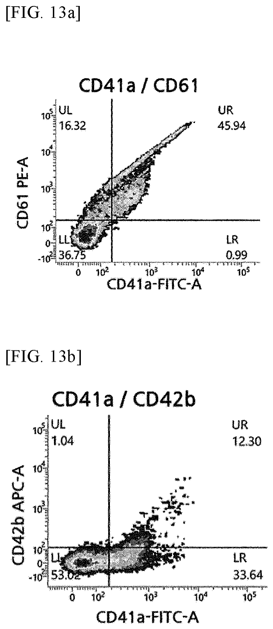

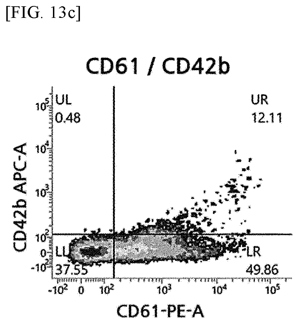

As a result, the material obtained from Floating Cell Preparative Example 1 was positive for the expression of platelet-specific markers (CD41a, CD42b, and CD61) (FIG. 12), and the material obtained from Floating Cell Preparative Example 3 was also positive for the expression of platelet-specific markers (CD41a, CD42b, and CD61) (FIG. 13). Through this, it was confirmed that the cell populations of Floating Cell Preparative Examples 1 and 3 were capable of producing platelets.

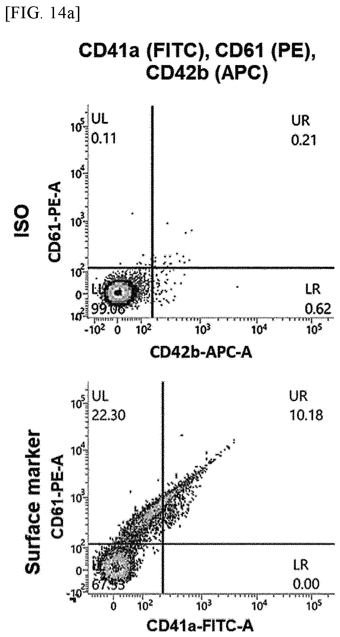

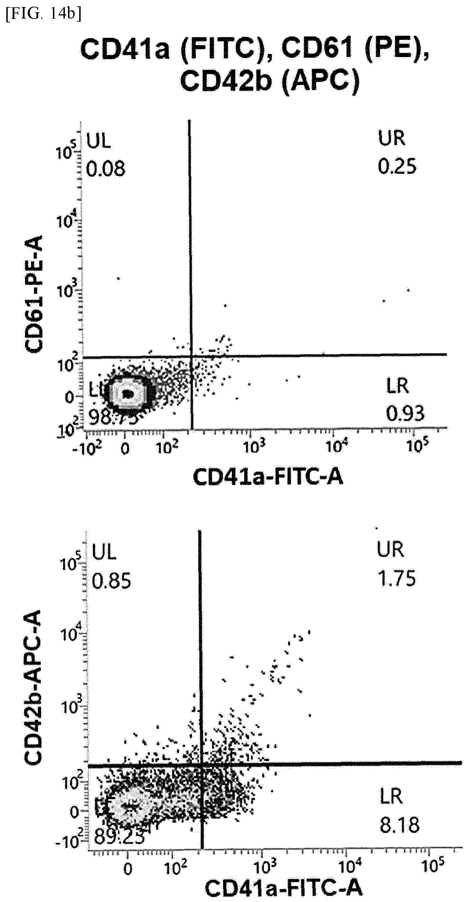

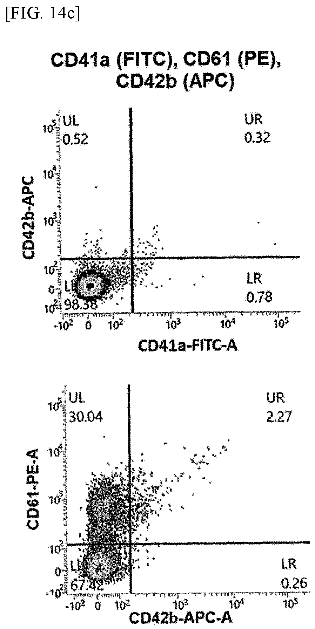

Meanwhile, the material obtained from Floating Cell Comparative Preparative Example 1 showed very low expression of CD41a and CD61, and in particular, CD42b showed expression close to negative (FIG. 14). Through this, it was confirmed that the material obtained from Comparative Preparative Example 1 did not show the surface marker characteristics of platelets, and that the cell population of Comparative Preparative Example 1 hardly differentiated into megakaryocytes, so platelets were not produced.

### (2) Confirmation of platelet function through ADP treatment

To confirm whether the materials obtained from Floating Cell Preparative Examples 1 and 3 are platelets with normal activity, the following experiments were performed.

The materials obtained from Floating Cell Preparative Examples 1 and 3 were centrifuged to remove precipitated megakaryocytes, and the suspended platelets were centrifuged again at 2000×g for 10 minutes. To collect the precipitated platelets due to centrifugation, all supernatant was removed and the platelets were resuspended in a small amount of PBS.

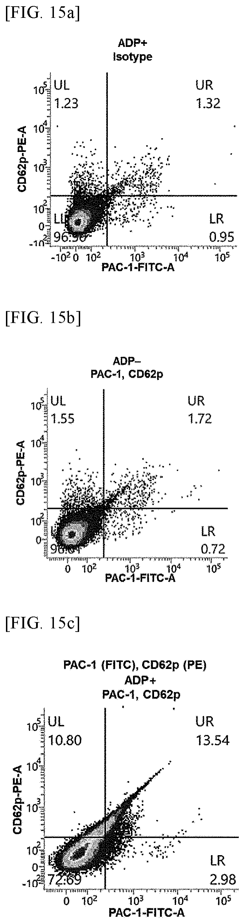

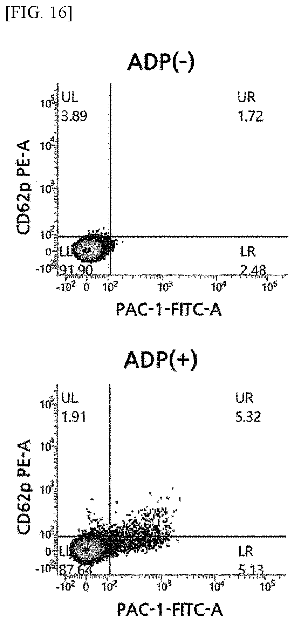

For PAC-1 analysis, platelets were treated with 100 µM ADP, reacted at room temperature for 15 minutes, and reacted with BD's anti-PAC-1-FITC and anti-CD62p-PE antibodies for 30 minutes. The reacted platelets were immediately subjected to FACS analysis. The materials obtained from Preparative Examples 1 and 3 were activated by ADP treatment, and the expression of PAC-1 and CD62p increased (FIGs. 15 and 16), confirming that the materials obtained from Preparative Examples 1 and 3 were platelets with normal activity. PAC-1 is a complex of CD41a and CD61 that appears when platelets are activated. CD62p (p-selectin) is a surface molecule whose expression increases when platelets are activated, which serves as a site where leukocytes can bind to platelets.

Through the experimental results described above, it was confirmed that, when the subsequent culture step is performed if the cell population satisfies a certain condition (the ratio of the number of cells expressing specific surface markers (e.g., CD41a, CD34, CD45) to the total number of cells is more than or less than a predetermined value) after culturing hiPSCs, the differentiation efficiency into megakaryocytes and the efficiency of platelet production could be increased.

### 4. Comparison of platelet production efficiency when only CD41a+ cells were selected and cultured subsequently

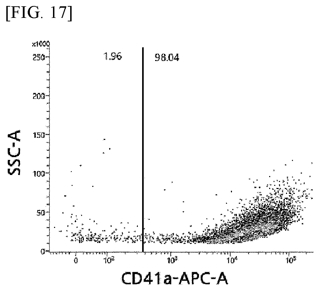

In the floating cell population obtained in the above Floating Cell Preparative Example 1, only CD41a+ cells were selected (FIG. 17) and subsequently cultured to compare the platelet production efficiency with that of the floating cell population in Floating Cell Preparative Example 1.

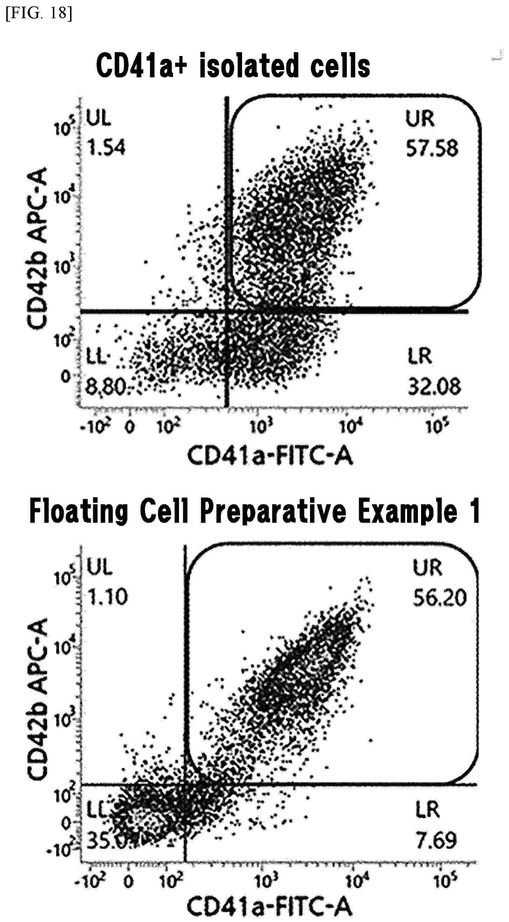

Using the same method as the FACS analysis in 3. (1) above, the expression of platelet-specific markers in the two cases was investigated and, as a result thereof, it was confirmed that the ratio of CD41a⁺/CD42b⁺ was similar, therefore, the platelet differentiation rates were similar (FIG. 18).

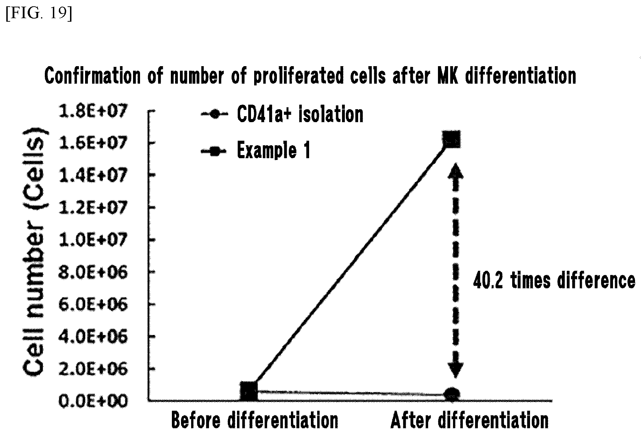

However, as a result of examining the cell proliferation ability in both cases, it was confirmed that in the case of differentiating a cell population including not only CD41a⁺ cells but also non-CD41a⁻ cells (e.g., CD41a⁻) into platelets, as in the Preparative Example 1, the cell proliferation ability was approximately 40 times higher than in the case of selecting CD41a⁺ and differentiating them into platelets (FIG. 19).

According to the above result, it was confirmed that, when a cell population further including CD41a⁻ cells is used for platelet production, as in Preparative Example 1 of the present invention, platelets can be produced more efficiently in terms of both quantity and quality, rather than by selecting only CD41a⁺ cells and using them for platelet production.

## Claims

1. A method for producing red blood cells, comprising:
(S1) culturing pluripotent stem cells to obtain a culture medium comprising hematopoietic stem cells;
(S2) obtaining a CD41⁻/CD34⁻ cell population from the culture medium; and
(S3) maturing the obtained cell population in a first medium comprising erythropoietin (EPO).

2. The method according to claim 1, further comprising:
proliferating the obtained cell population in a second medium comprising erythropoietin, stem cell factor, and interleukin-3.

3. The method according to claim 1, further comprising:
differentiating the obtained cells in a third medium comprising erythropoietin and stem cell factor.

4. The method according to claim 1, wherein the culture medium comprises red blood cell precursors and megakaryocyte precursors.

5. The method according to claim 1, wherein the maturation step is performed for 4 to 14 days.

6. The method according to claim 1, wherein (S1) comprises:
(S1a) culturing pluripotent stem cells in a fourth medium comprising a GSK3 inhibitor;
(S1b) culturing the cells cultured in the fourth medium in a fifth medium comprising vascular endothelial growth factor and basic fibroblast growth factor; and
(S1c) culturing the cells cultured in the fifth medium in a sixth medium comprising vascular endothelial growth factor, basic fibroblast growth factor, and a transforming growth factor beta signaling inhibitor.

7. The method according to claim 1, further comprising:
(S0) seeding pluripotent stem cells at a density of 2,000 to 20,000 cells/cm² on the bottom of a culture vessel.

8. The method according to claim 1, wherein the pluripotent stem cells comprise human induced pluripotent stem cells.

9. A method for producing a blood product, comprising a process of mixing red blood cells produced by any one of the methods 1 to 8 above with other blood components.

10. A method for producing red blood cells and platelets, comprising:
(S1) culturing pluripotent stem cells to obtain a culture medium comprising hematopoietic stem cells;
(S2) sorting a CD41⁻/CD34⁻ cell population and a CD41a⁺ or CD34⁺ cell population from the culture medium;
(S3) maturing the sorted CD41⁻/CD34⁻ cell population in a first medium comprising erythropoietin; and
(S4) differentiating the sorted CD41a⁺ or CD34⁺ cell population into megakaryocytes in a seventh medium.

11. The method according to claim 10, further comprising:
proliferating the sorted CD41⁻/CD34⁻ cell population in a second medium comprising erythropoietin, stem cell factor and interleukin-3.

12. The method according to claim 10, further comprising:
differentiating the sorted CD41⁻/CD34⁻ cell population in a third medium comprising erythropoietin and stem cell factor.

13. The method according to claim 10, wherein the culture medium comprises red blood cell precursors and megakaryocyte precursors.

14. The method according to claim 10, wherein (S3) is performed for 4 to 14 days.

15. The method according to claim 10, wherein (S1) comprises:
(S1a) culturing the pluripotent stem cells in a fourth medium comprising a GSK3 inhibitor;
(S1b) culturing the cells cultured in the fourth medium in a fifth medium comprising vascular endothelial growth factor and basic fibroblast growth factor; and
(S1c) culturing the cells cultured in the fifth medium in a sixth medium comprising vascular endothelial growth factor, basic fibroblast growth factor, and a transforming growth factor beta signaling inhibitor.

16. The method according to claim 10, further comprising:
seeding the pluripotent stem cells (S0) at a density of 2,000 to 20,000 cells/cm² on the bottom of a culture vessel.

17. The method according to claim 10, further comprising:
culturing and maturing megakaryocytes in an eighth medium comprising thrombopoietin.

18. The method according to claim 10, wherein the seventh medium comprises thrombopoietin, stem cell factor, interleukin-3 and interleukin-6.

19. The method according to claim 10, wherein the pluripotent stem cells comprise human induced pluripotent stem cells.

20. A method for producing a blood product, comprising mixing red blood cells and platelets produced by the method according to any one of claims 10 to 19.
